# FASCICULE DE BREVET EUROPEEN

(11) **EP 1 177 810 B1**
(45) Date de publication et mention de la délivrance du brevet: **24.03.2004**
(21) Numéro de dépôt: 01402065.5
(22) Date de dépôt: 31.07.2001
(51) Int. Cl.: A61M 16/20

(54) **Appareil d'aide à la ventilation d'un patient**
Unterstützungsvorrichtung zur Beatmung eines Patienten
Apparatus for providing ventilation support to a patient

(30) Priorité: 31.07.2000 FR 0010036
(43) Date de publication de la demande: 06.02.2002
(73) Titulaire: Société d'Applications Industrielles Medicales et Electroniques ( SAIME), 77176 Savigny Le Temple (FR)
(72) Inventeur: Chalvignac, Philippe, 77760 Achères-la-Forêt (FR)
(74) Mandataire: Martin, Jean-Jacques

(56) Documents cités:
- EP-A- 0 621 052
- WO-A-96/11717
- WO-A-97/10018
- GB-A- 2 198 812
- US-A- 3 972 327
- US-A- 5 370 112

## Description

L'invention concerne un appareil d'aide à la ventilation d'un patient.

L'invention concerne plus particulièrement un appareil d'aide à la ventilation d'un patient qui respire par des cycles successifs dont chacun comporte une phase inspiratoire et une phase expiratoire.

Il existe de nombreux types d'appareils d'aide à la ventilation, aussi appelés respirateurs, qui permettent de pallier les troubles de la respiration en procurant notamment au patient une aide insufflatoire lors des phases inspiratoires.

Le patient porte généralement sur le visage un masque qui recouvre le nez et la bouche et auquel le circuit inspiratoire est relié de manière notamment à forcer l'inspiration du patient.

De façon à pouvoir être utilisé pour plusieurs types de pathologies, l'appareil d'aide à la ventilation doit pouvoir fonctionner selon des modes d'aide à la ventilation de types différents, notamment un mode du type ventilation volumétrique, un mode du type ventilation à percussions et un mode du type ventilation barométrique.

Selon le mode d'aide à la ventilation du type volumétrique, l'appareil d'assistance doit transmettre au patient un volume d'air constant et/ou déterminé. Le débit d'air fourni au patient au cours de la phase inspiratoire peut être constant, décroissant, croissant, sinusoïdal, etc...

La valeur du débit à transmettre au patient peut varier dans une large plage de valeurs qui peut s'étendre de 5 à 150 litres par minute. Dans tous les cas, il doit être parfaitement maîtrisé. De plus, la durée de l'augmentation du débit lors du passage de la phase expiratoire à la phase inspiratoire du patient, ainsi que la durée de la diminution du débit lors du passage de la phase inspiratoire à la phase expiratoire du patient doivent être les plus courtes possibles.

Selon le mode d'aide à la ventilation du type barométrique, l'appareil d'assistance doit transmettre au patient du gaz, et notamment un volume de gaz, sous une pression constante durant toute la phase inspiratoire du patient. Selon ce type de ventilation, le débit du gaz sous pression est plus important que dans le mode du type ventilation volumétrique. La durée de montée en pression du gaz doit être la plus courte possible. Or, les appareils d'aide à la ventilation, notamment les appareils de ventilation à domicile, permettent difficilement de réduire cette durée au minimum.

Selon le troisième mode d'aide à la ventilation à percussions, l'appareil d'aide à la ventilation doit transmettre au patient un débit de gaz qui est modulé en amplitude au cours des différentes phases de la ventilation. La fréquence des oscillations, qui peut être comprise entre 1 et 10 coups par seconde, est difficilement obtenue par les appareils d'aide du type connu. En effet, l'inertie des valves inspiratoires ne permet pas toujours de satisfaire les exigences décrites précédemment.

De plus, l'appareil d'aide à la ventilation nécessite une alimentation électrique pour permettre le fonctionnement d'éléments tels que la source de gaz sous pression qui peut être un soufflet ou une turbine.

Les appareils de ventilation, notamment les appareils de ventilation à domicile, ne permettent pas toujours l'utilisation des trois modes précités.

Un tel appareil peut être utilisé en milieu hospitalier ou au domicile des patients. Dans un cas comme dans l'autre, il peut être utilisé par des patients pouvant se déplacer, de façon à ne pas entraver leur mobilité, et il est alors intéressant de leur proposer des appareils d'aide autonomes à la ventilation.

Dans ce cas, il est nécessaire d'équiper les appareils avec des batteries d'accumulateurs électriques. Cependant, l'autonomie est fortement limitée à cause de l'encombrement, du poids, ainsi que du coût de tels accumulateurs qu'il est nécessaire d'intégrer à l'appareil d'aide à la ventilation.

De plus, la très large diversité des pathologies traitées par les appareils d'aide à la ventilation nécessite une certaine spécialisation de chaque appareil. Ainsi, à chaque type d'appareil correspond une catégorie de pathologies. Ceci diminue le nombre d'appareils produits pour chaque catégorie et augmente les coûts d'études et de fabrication

Dans le but de remédier à ces inconvénients, l'invention propose un appareil d'aide à la ventilation d'un patient qui respire par des cycles successifs dont chacun comporte une phase inspiratoire et une phase expiratoire, du type qui comporte :
- une source de gaz sous pression dont un orifice de sortie fournit un flux de gaz sous pression destiné à être transmis aux voies aériennes supérieures du patient ;
- un bloc de distribution du flux de gaz sous pression qui comprend un circuit de transmission qui relie l'orifice de sortie de la source de gaz à une première extrémité libre d'une conduite principale inspiratoire sur la seconde extrémité libre de laquelle est fixé un masque, notamment du type facial, destiné à être porté par le patient ; et
- une valve inspiratoire de régulation dudit flux de gaz qui est interposée dans le circuit de transmission et qui est commandée par un circuit de commande de l'appareil, en fonction notamment des valeurs du débit et de la pression du gaz dans la conduite principale,
caractérisé en ce que la valve inspiratoire est réalisée sous la forme d'un distributeur rotatif.

Selon d'autres caractéristiques de l'invention :
- la valve inspiratoire de régulation du flux de gaz comporte un corps de valve tubulaire comportant, dans sa paroi, une ouverture oblongue longitudinale qui permet la transmission du flux de gaz depuis l'orifice de sortie de la source de gaz à la première extrémité libre de la conduite principale inspiratoire et compose, un boisseau qui est monté mobile en rotation dans le corps, qui est fermé à l'une de ses extrémités axiales et dont un bord d'extrémité axiale opposé à son extrémité fermée est profilé de façon à provoquer, lors de la rotation du boisseau, la variation progressive de la section de passage de l'ouverture oblongue permettant la transmission du flux de gaz ;
- l'orifice de sortie de la source de gaz débouche dans la première extrémité libre d'un premier trou dont la seconde extrémité libre est obturée par l'extrémité fermée du boisseau, et le bloc de distribution comporte un évidement qui débouche d'une part en vis-à-vis de l'ouverture oblongue longitudinale de laquelle il est complémentaire et, d'autre part, dans un deuxième trou borgne sur l'extrémité libre duquel est fixée la première extrémité libre de la conduite principale inspiratoire ;
- le boisseau est entraîné en rotation par un moteur, notamment du type pas à pas, commandé par le circuit de commande de l'appareil ;
- le boisseau comporte un dispositif d'indexation de sa position angulaire ;
- lorsque l'appareil fonctionne en mode d'assistance respiratoire par ventilation volumétrique, au cours de la phase inspiratoire duquel l'appareil doit fournir au patient un volume de gaz prédéterminé, le circuit de commande de l'appareil commande la position angulaire du boisseau et la pression fournie par la source de gaz de façon que la section de passage permette la transmission au patient du volume de gaz prédéterminé ;
- lorsque l'appareil fonctionne en mode d'assistance respiratoire par ventilation barométrique, au cours de la phase inspiratoire duquel l'appareil doit fournir au patient un gaz sous une pression prédéterminée, le circuit de commande de l'appareil commande la position angulaire du boisseau de façon que la section de passage corresponde à la totalité de l'ouverture oblongue et le circuit de commande de l'appareil commande la pression fournie par la source de gaz, pour que la pression du gaz dans la conduite inspiratoire soit la pression de gaz prédéterminée ;
- lorsque l'appareil fonctionne en mode d'assistance par ventilation à percussions, au cours de la phase inspiratoire duquel l'appareil doit fournir au patient un débit oscillant autour d'un débit de gaz prédéterminé, le circuit de commande de l'appareil commande l'oscillation de la position angulaire du boisseau autour d'une position prédéterminée correspondant au débit prédéterminé et commande la pression fournie par la source de gaz ;
- la source de gaz sous pression comporte une machine électrique tournante, pilotée en vitesse par le circuit de commande, dont une extrémité libre du rotor entraîne en rotation une roue à aubes qui entraîne le flux gazeux dans une volute de guidage d'un carter, lorsque la machine électrique tournante est alimentée électriquement ;
- le carter comporte au moins un point d'injection, débouchant dans la volute, d'au moins un gaz comprimé qui est fourni par une autre source de pression amont, sous une seconde pression supérieure à la première pression, et le gaz comprimé sous la seconde pression est susceptible d'entraîner en rotation la roue à aubes ainsi que le rotor de la machine électrique tournante, de façon que le gaz sous pression soit fourni au patient sous la première pression et que la machine électrique tournante fonctionne en génératrice d'énergie électrique et produise de l'énergie électrique ;
- le flux d'au moins un gaz sous une seconde pression est injecté dans le carter selon une direction sensiblement tangentielle à la volute ;
- la source de pression amont consiste au moins partiellement en un circuit de gaz sous pression, disponible en milieu hospitalier ;
- la source de pression comporte un réservoir de gaz sous pression ;
- le réservoir est intégré à l'appareil d'assistance ;
- le gaz sous la seconde pression est composé au moins partiellement d'air ;
- le gaz comprimé sous la seconde pression est composé au moins partiellement d'un gaz thérapeutique ;
- le gaz thérapeutique est de l'oxygène ;
- des moyens de régulation de pression sont interposés entre ladite autre source de pression amont et le point d'injection ;
- l'appareil comporte une valve de dosage du gaz comprimé sous la seconde pression, dont l'ouverture est commandée de façon proportionnelle à l'ouverture de la valve inspiratoire ;
- l'énergie électrique fournie par la machine électrique tournante, fonctionnant en génératrice, alimente au moins partiellement les systèmes consommateurs d'électricité de l'appareil ;
- l'énergie électrique produite par la machine électrique tournante, fonctionnant en génératrice, alimente et charge au moins partiellement une batterie d'accumulateurs de l'appareil ;
- l'énergie électrique produite par la machine électrique tournante est supérieure à l'énergie électrique consommée par les systèmes consommateurs d'électricité, de façon que l'appareil d'aide à la ventilation respiratoire soit autonome ;
- le bloc de distribution comporte des moyens de pilotage qui permettent de piloter l'ouverture et la fermeture d'une valve expiratoire qui est agencée indifféremment dans la conduite inspiratoire d'un circuit simple ou dans la conduite expiratoire d'un circuit double ;
- lorsque l'appareil comporte un circuit double, la vanne expiratoire est agencée dans un module qui est fixé sur le bloc de distribution et qui comporte un conduit de façon à relier les moyens de pilotage à la vanne expiratoire ;
- les moyens de pilotage permettent d'appliquer à la valve expiratoire une pression expiratoire positive, et la pression expiratoire positive est fournie par un ventilateur ;
- le bloc de distribution comporte des moyens de mesure qui déterminent le débit du gaz qui circule, lors de la phase expiratoire, dans la conduite inspiratoire d'un circuit simple, ou dans la conduite expiratoire d'un circuit double ;
- lorsque l'appareil comporte un circuit double, il comprend un module qui est fixé sur le bloc de distribution et qui comporte au moins un conduit qui relie les moyens de mesure à la conduite expiratoire.

D'autres caractéristiques et avantages de l'invention apparaîtront à la lecture de description détaillée qui suit pour la compréhension de laquelle on se reportera aux dessins annexés parmi lesquelles :
- la figure 1 est une vue schématique et simplifiée d'un appareil d'aide à la ventilation réalisé selon l'état de la technique, l'appareil étant illustré lors d'une phase inspiratoire ;
- la figure 2 est une vue schématique et simplifiée d'une partie d'un appareil d'aide à la ventilation réalisé conformément aux enseignements de l'invention ;

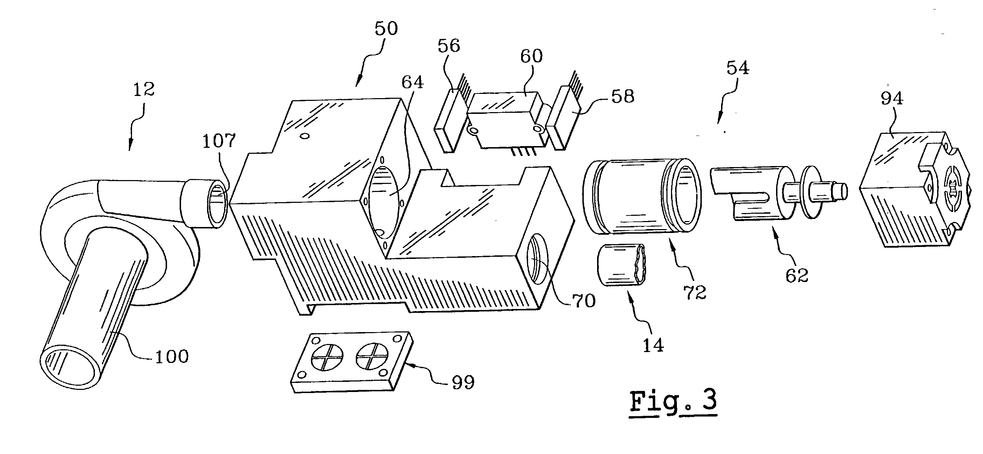

- la figure 3 est une vue en perspective éclatée qui représente principalement le bloc de distribution du flux de gaz sous pression, réalisé conformément à l'invention, ainsi que la source de gaz sous pression ;

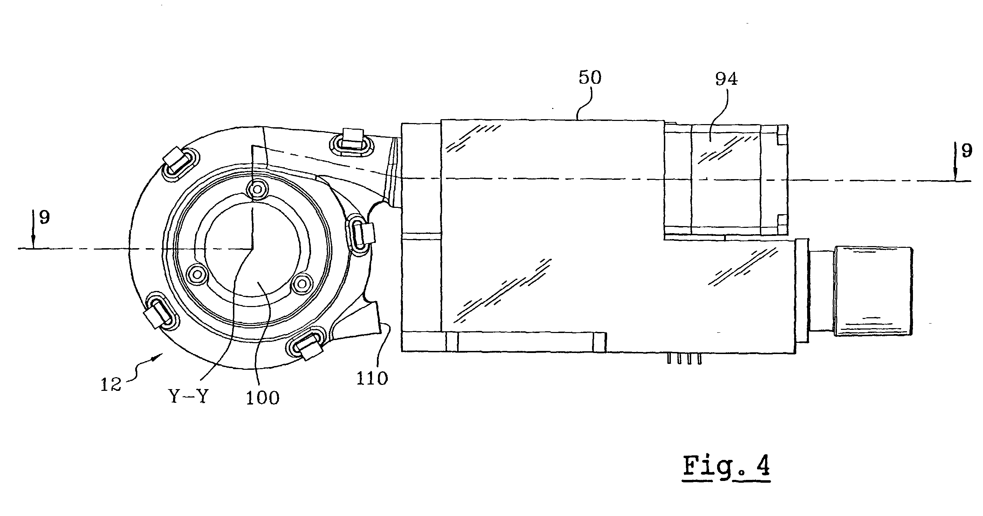

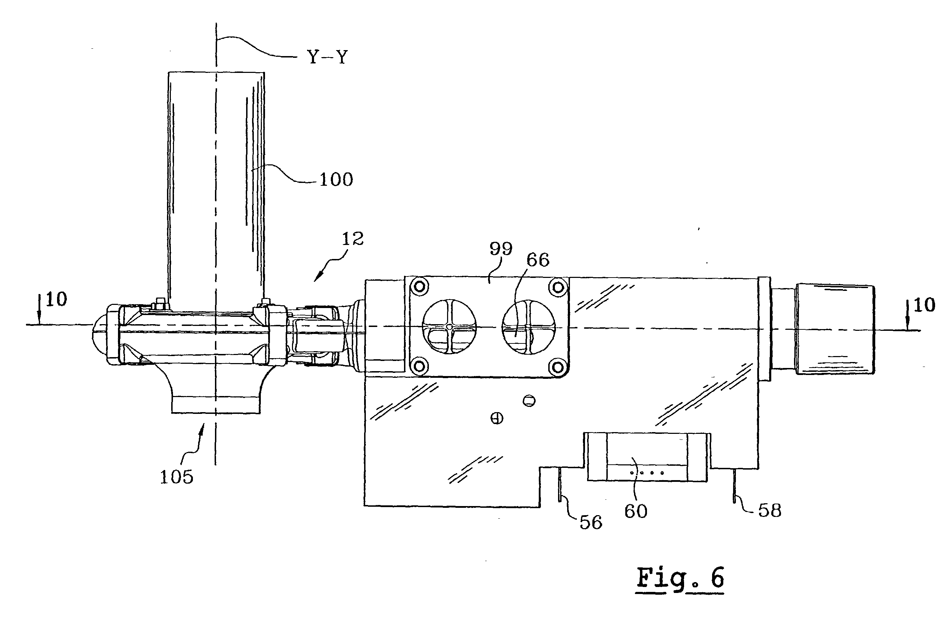

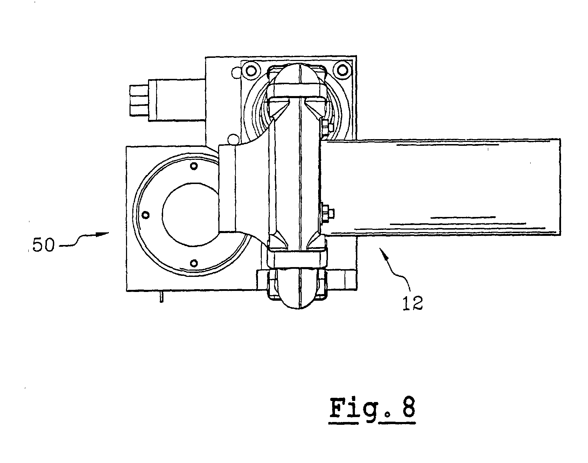

- les figures 4 à 8 représentent les différentes vues extérieures la source de gaz sous pression assemblée avec le bloc de distribution du flux de gaz sous pression, cet ensemble étant illustré respectivement en vue de face, en vue de dessus, en vue de dessous et en vues de côté ;
- la figure 9 est une vue en coupe de la source de gaz sous pression et du bloc de distribution du flux de gaz, selon la ligne 9-9 de la figure 4 ;
- la figure 10 est une vue en coupe partielle de la source de gaz sous pression et du bloc de distribution du flux de gaz selon la ligne 10-10 de la figure 6 ;
- la figure 11 est une vue schématique développée du boisseau rotatif de la valve inspiratoire du bloc de distribution ;
- la figure 12 est une vue partielle en détail de celle représentée à la figure 9, la valve inspiratoire étant dans une position fermée ;
- la figure 13 est une vue similaire à celle représentée à la figure précédente, la valve d'inspiration étant dans une position partiellement ouverte ;
- la figure 14 est une vue similaire à celle représentée à la figure 2, l'appareil d'aide à la ventilation étant équipé d'un circuit simple ;
- la figure 15 est une vue similaire à celle représentée à la figure 2, l'appareil d'aide à la ventilation étant équipé d'un circuit double.

Dans la suite de la description, les éléments identiques ou similaires seront désignés par les mêmes chiffres de référence.

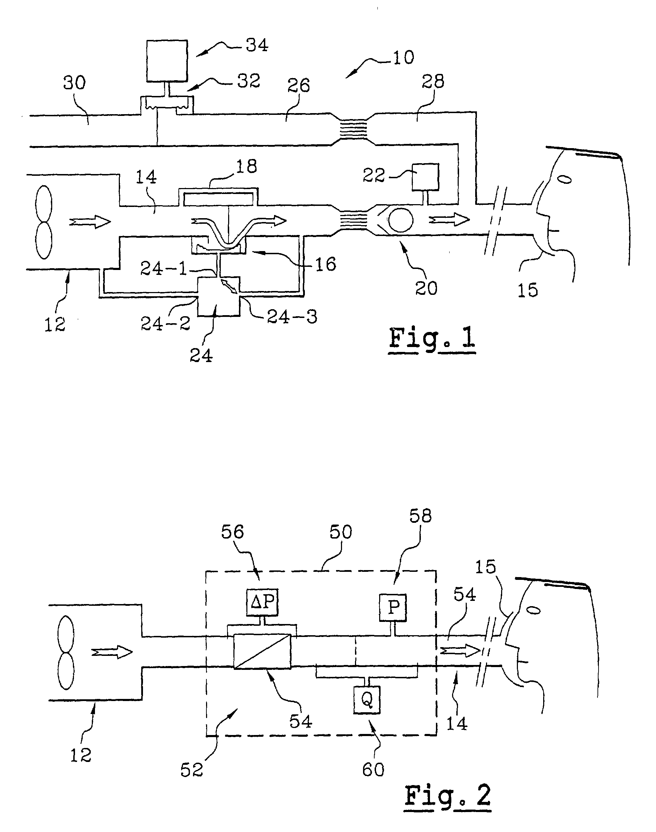

On a illustré sur la figure 1 un appareil d'aide à la ventilation d'un patient réalisé selon l'état de la technique.

L'appareil 10 comporte tout d'abord une source d'air sous pression 12 qui est par exemple réalisée sous la forme d'un motoventilateur ou d'une turbine, à moteur électrique, qui fournit en permanence de l'air sous pression à une conduite inspiratoire principale 14 dont une extrémité aval est par exemple reliée à un masque 15 qui recouvre les voies aériennes supérieures du patient, c'est-à-dire le nez et la bouche.

Dans la conduite inspiratoire 14, on a interposé une valve inspiratoire 16 qui est ici réalisée sous la forme d'une valve ballon à commande pneumatique. La valve inspiratoire 16 est ouverte pendant les phases inspiratoires du patient, et elle est fermée pendant les phases expiratoires. Toutefois, une conduite de dérivation 18 contourne la valve inspiratoire 16 de manière à permettre le passage dans la conduite inspiratoire 14, même pendant les phases expiratoires, d'un léger débit de fuite qui permet par exemple de compenser des fuites d'air au niveau du masque 15 du patient.

En aval de la valve inspiratoire 16, on trouve successivement dans la conduite inspiratoire 14 un clapet anti-retour 20, qui empêche que du gaz expiré par le patient ne puisse remonter en direction de la source d'air sous pression, et un capteur de débit et de pression 22.

Pour la commande de la valve inspiratoire 16, on peut voir qu'il est prévu une électrovanne 24 qui possède trois entrées 24-1, 24-2 et 24-3 dont chacune est reliée par un tuyau respectivement au ballon de la valve inspiratoire 16, à la sortie de la source d'air sous pression 12, et à la conduite inspiratoire 14 en aval de la valve inspiratoire 16.

L'électrovanne 24 de commande de la valve inspiratoire 16 peut donc mettre en communication soit son entrée 24-1 avec son entrée 24-2, soit son entrée 24-1 avec son entrée 24-3. Dans le premier cas, la pression qui règne dans le ballon de la valve inspiratoire 16 est alors celle fournie par la source de pression 12, ce qui provoque la fermeture de la valve inspiratoire 16. Au contraire, lorsque l'électrovanne 24 met en communication ses entrées 24-1 et 24-3, conformément à la figure 1, la valve inspiratoire 16 s'ouvre et permet à l'air ou au mélange sous pression fourni par la source de pression 12 d'être acheminé jusqu'au patient.

Pour plus de détails concernant le fonctionnement du circuit inspiratoire, on se reportera avec profit au document FR-A-2.760.196 qui décrit une valve inspiratoire 16 de même type.

L'appareil d'aide à la respiration 10 comporte par ailleurs une conduite expiratoire 26 dont une extrémité amont 28 est par exemple reliée au masque 15 du patient et dont une extrémité aval 30 débouche par exemple dans l'atmosphère.

De manière connue, une valve expiratoire 32, réalisée sous la forme d'une valve ballon, est interposée dans la conduite expiratoire 26. La valve expiratoire 32 est fermée pendant les phases inspiratoires du patient, et elle est ouverte, partiellement ou totalement, lors des phases expiratoires. La pression régnant dans le ballon de la valve d'expiration 32, qui détermine l'ouverture ou la fermeture de la valve 32, est commandée par un circuit de commande 34.

Le fonctionnement du circuit de commande 34 de la valve expiratoire 32 va maintenant être décrit plus en détail.

Lors d'une phase inspiratoire, le ballon de la valve expiratoire 32 est gonflé. Ainsi, la valve expiratoire 32 est fermée, empêchant ainsi toute circulation de gaz dans la conduite expiratoire 26.

En début de phase expiratoire, le ballon de la valve expiratoire 32 est dégonflé de façon à permettre la circulation de gaz dans la conduite expiratoire 26.

Lorsque l'on souhaite imposer au patient une pression d'expiration positive, on est alors amené à provoquer, relativement rapidement après le début de la phase expiratoire et par exemple après une durée comprise entre 100 et 200 millisecondes, l'arrêt du dégonflage du ballon de la valve expiratoire 32.

On peut par exemple choisir d'arrêter le dégonflage du ballon de la valve expiratoire 32 lorsque la pression dans le ballon de la valve 32 a chuté jusqu'à une valeur supérieure de seulement 3 millibars à la valeur choisie pour réaliser la pression d'expiration positive.

Ainsi, la valve expiratoire 32 ne s'ouvre que lorsque l'effort exercé par l'air expiré par le patient, en amont de la valve expiratoire 32 dans la conduite expiratoire 26, devient supérieur à une valeur de seuil que l'on peut régler en ajustant la pression du ballon de la valve expiratoire 32.

Cependant, un tel type d'appareil d'aide à la ventilation 10 ne permet pas un fonctionnement optimal tel que décrit précédemment.

On connaît aussi des appareils d'aide à la ventilation portatifs, tel que celui décrit et représenté dans le document WO-A-96/11717. Ce document décrit notamment un appareil unique qui a la capacité de s'adapter aux différents modes d'aide à la ventilation précédemment décrits. Cette solution prévoit de faire varier le débit d'air en agissant directement sur la vitesse de rotation d'un rotor de compresseur constituant la source d'air comprimé.

On connaît aussi des appareils d'aide à la ventilation tel que celui décrit et représenté par le document US-A-3.972.327. La variation du débit d'air nécessaire lors des différents modes d'aide à la ventilation est effectuée par une vanne qui est interposée dans le circuit d'admission.

Cette vanne est notamment constituée d'un corps tubulaire comportant, dans sa paroi, une ouverture qui permet la transmission du flux de gaz depuis l'orifice de sortie de la source de gaz à la première extrémité libre de la conduite principale inspiratoire, et elle comporte un boisseau qui est monté mobile en rotation dans le corps tubulaire, qui est fermé à l'une de ses extrémités axiales et qui comporte un trou réalisé dans sa paroi axiale de façon à provoquer, lors de la rotation du boisseau, la variation progressive de la section de passage de l'ouverture permettant la transmission du flux de gaz. Ainsi, le système décrit dans ce document permet de faire varier le débit de zéro au maximum en faisant tourner le boisseau de quelques degrés autour de son axe longitudinal.

De plus, étant donné la section circulaire de l'orifice du corps de valve et du trou du boisseau, la variation de la section de passage de l'ouverture n'est pas linéaire.

L'invention propose que l'appareil d'aide à la ventilation 10 du patient comporte un bloc de distribution 50 du flux du gaz sous pression qui comprend un circuit de transmission 52 qui relie l'orifice de sortie de la source de gaz 12 sous pression à une première extrémité libre 54 de la conduite inspiratoire principale 14 sur la seconde extrémité de laquelle est fixé le masque 15 destiné à être porté par le patient.

Un tel appareil d'aide à la ventilation 10 est représenté à la figure 2.

De façon à réguler et à moduler de façon précise le flux de gaz entre la source de gaz 12 sous pression et la conduite inspiratoire 14, une valve inspiratoire 54 est interposée dans le circuit de transmission 52.

La valve inspiratoire 54 est commandée par un circuit de commande, non représenté, de l'appareil, notamment en fonction des valeurs du débit et de la pression du gaz dans le circuit de transmission 52. Le circuit de commande comporte des circuits électroniques qui permettent notamment le traitement ainsi de l'élaboration de signaux électriques.

À cet effet, des premiers moyens de mesure 56 permettent de déterminer la perte de charge provoquée par la valve inspiratoire 54. Les premiers moyens de mesure 56 transmettent au circuit de commande une information représentative de la différence de pression entre l'amont et aval de la valve inspiratoire 54.

Les moyens de mesure 56 peuvent consister en un capteur de pression différentielle qui est fixé sur le bloc de distribution 50, et qui est relié au circuit de transmission 52 par un premier canal et un second canal ou passage, non représentés, en amont et en aval de la valve inspiratoire 54 respectivement.

Des deuxièmes moyens de mesure 58 permettent de déterminer la pression qui règne à proximité de l'extrémité aval du circuit de transmission 52. Les deuxièmes moyens de mesure 58 transmettent au circuit de commande une information représentative de la pression dans la conduite inspiratoire 14.

Les deuxièmes moyens de mesure 58 peuvent consister en un capteur de pression qui est relié au circuit de transmission 52.

Ils peuvent aussi consister en un capteur de pression différentielle similaire à celui utilisé pour les premiers moyens pression 56. L'utilisation d'un tel capteur permet de diminuer le nombre de composant de l'appareil d'aide à la ventilation 10, et par conséquent de réduire son coût de fabrication. Dans ce cas, le capteur de pression différentielle comporte deux prises de pression, l'une étant reliée, par un canal non représenté, au circuit de transmission 52, l'autre capteur étant relié directement à l'atmosphère.

Des troisièmes moyens de mesure 60 permettent de déterminer le débit du flux de gaz qui circule dans le circuit de transmission 52 et qui correspond sensiblement au flux de gaz qui est transmis au masque 15 du patient.

Conformément à la figure 2, les moyens de mesure 60 comportent deux capteurs de pression qui sont situés de part et d'autre d'un organe qui a pour fonction de provoquer une chute de pression.

Conformément aux enseignements de l'invention et de façon à réguler de façon optimale le flux de gaz qui circule dans le circuit de transmission 52, la valve inspiratoire 54 est du type à boisseau rotatif.

Elle permet de faire varier la section de passage du flux de gaz qui la traverse par la rotation d'un organe mobile entraîné par un dispositif d'entraînement rapide et présentant une faible inertie, de façon à réduire, notamment les temps d'ouverture et de fermeture de la valve, lors des passages de la phase inspiratoire à la phase expiratoire et inversement.

La valve inspiratoire 54 se présente sous la forme d'un distributeur rotatif qui comporte alors un corps tubulaire 62 qui est aligné axialement avec l'orifice de sortie de la source de gaz 12 d'axe X-X.

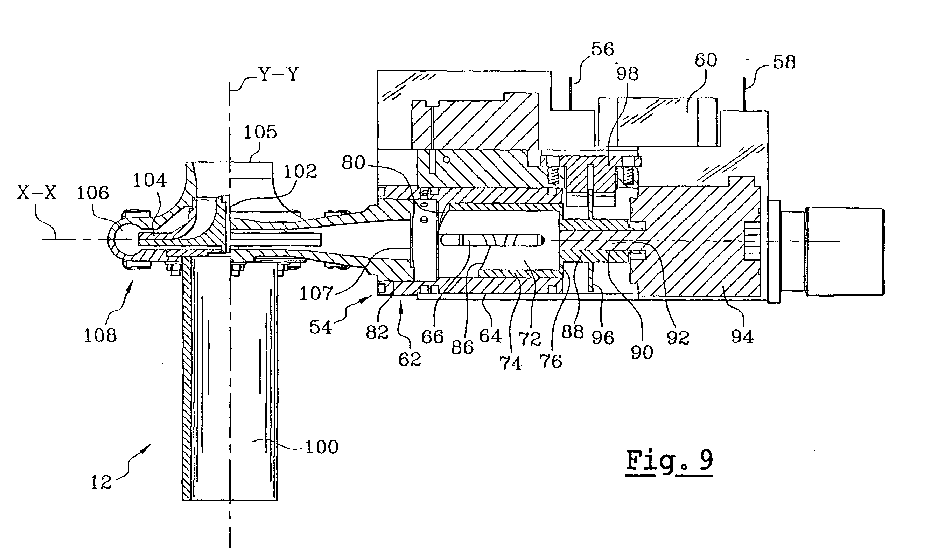

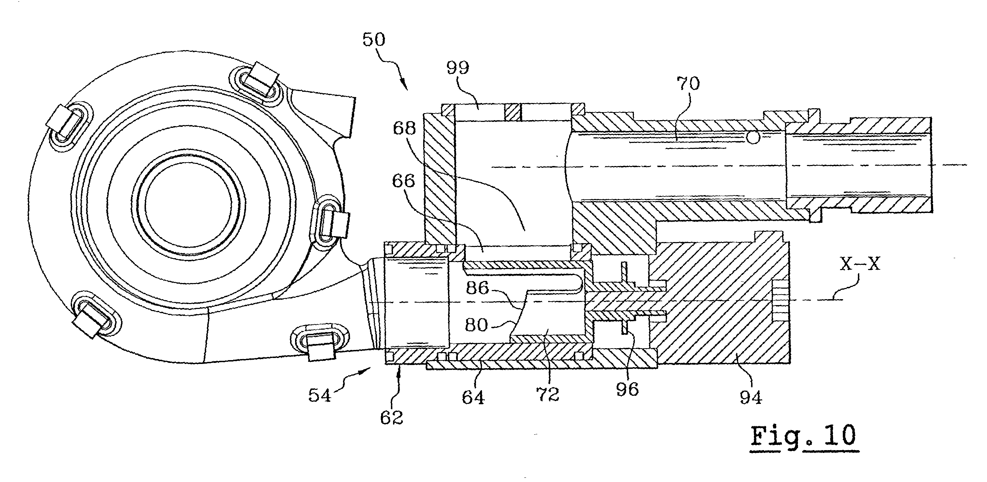

Conformément aux figures 9 et 10, le corps tubulaire 62 est reçu de façon étanche dans un premier trou 64 du bloc de distribution 50.

Le corps tubulaire 62 comporte, dans sa paroi cylindrique annulaire, une première ouverture oblongue, ou lumière 66, d'orientation axiale. Un évidement 68 est réalisé dans le bloc de distribution 50, en vis-à-vis de l'ouverture oblongue 66.

L'ouverture oblongue 66 et l'évidement 68 permettent ainsi de relier l'intérieur du corps tubulaire 62 et un deuxième trou 70 réalisé axialement dans le bloc de distribution 50.

Conformément à la figure 10, les deux trous 64 et 70 sont parallèles et superposés l'un à l'autre.

L'ouverture oblongue 66 définit la section maximale de passage du flux de gaz à travers la valve inspiratoire 54. La section de passage maximale ainsi définie est supérieure à la section de passage nécessaire à la transmission du volume de gaz maximal, lorsque l'appareil d'aide à la ventilation 10 fonctionne en mode d'assistance respiratoire par ventilation volumétrique.

De façon à gérer le débit de gaz sous pression traversant la valve 54, c'est-à-dire le débit du gaz qui est transmis aux voies aériennes du patient, un boisseau 72 est monté mobile en rotation autour de l'axe X-X.

Le boisseau mobile 72 comporte une jupe ou chemise 74 qui s'étend axialement depuis une paroi transversale arrière 76 permettant d'obturer l'extrémité aval 78 du corps tubulaire 62. Ainsi, le flux d'air sous pression introduit dans le premier trou 64 ne peut s'en échapper que par l'ouverture oblongue 66 et l'évidement 68.

La jupe 74 s'étend ainsi axialement de la paroi transversale arrière 76 vers l'extrémité amont du premier depuis le trou 64. Sa dimension axiale maximale est définie de façon qu'une partie avant de la jupe 74 puisse, en position fermée, obturer totalement l'ouverture oblongue 66 du corps tubulaire 62.

De façon à simplifier la réalisation de l'étanchéité du premier trou 64, l'orifice de sortie de la source de gaz 12 est montée de façon étanche directement à l'intérieur d'un orifice 82 de l'extrémité libre amont du corps tubulaire 62.

Le bord d'extrémité axiale avant 80 de la jupe 74, qui est opposée à la paroi transversale 76, est profilé.

En général, le profil du bord d'extrémité axiale 80 comporte deux zones.

Une première zone consiste en une rainure axiale 84 qui s'étend jusqu'à proximité de la paroi transversale arrière 76 du boisseau 72, de façon que, lorsque le boisseau 72 est dans une position angulaire dite de pleine ouverture, la rainure axiale 84 s'étend en vis-à-vis de l'ouverture oblongue 66 de manière que la section de passage du flux corresponde à la section de passage maximale définie précédemment. Ainsi, la largeur de la rainure axiale 84 peut correspondre à un secteur d'angle d'une vingtaine de degrés.

La seconde zone correspond à une courbe en forme générale de spirale 86 qui s'étend entre les deux bords axiaux parallèles de la rainure 84.

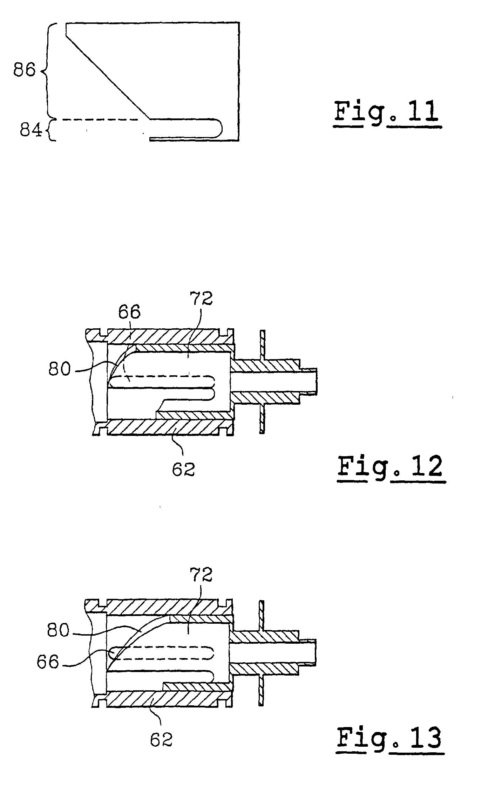

La figure 11 représente un exemple de développé de la jupe 74 faisant plus clairement apparaître les deux zones et notamment le profil de la spirale 86.

Ainsi, la rotation du boisseau provoque une obturation de l'ouverture oblongue 66 de façon à faire varier la section de passage du flux de gaz.

Le boisseau 72 comporte une queue 88 qui s'étend axialement depuis la paroi transversale 76, dans une direction arrière opposée à la source de gaz 12 sous pression. Un trou 90 d'axe X-X est réalisé dans la queue 88 de façon à permettre le passage d'un organe 92 d'entraînement en rotation du boisseau 72. L'organe 92 est immobilisé en rotation par rapport à la queue 88. Avantageusement l'organe 92 est monté serré dans l'autre trou axiale 90.

L'organe 92, qui entraîne en rotation le boisseau 72, est ici l'arbre de sortie d'un moteur électrique 94 du type pas à pas. La rotation du moteur 94 est commandée par le circuit de commande de l'appareil 10.

De façon à indexer angulairement la position du boisseau 72, et donc celle de la spirale 86, par rapport à la première ouverture oblongue 66, un voile 96 transversal est agencé sur la queue 88. Le voile 96 comporte sur la périphérie de ses faces radiales des dispositifs permettant l'identification de sa position angulaire.

Le voile 96 peut alors correspondre à une roue de codage qui coopère avec un dispositif optique 98, partiellement représenté à la figure 9.

Ainsi, de manière générale connue, le dispositif optique 98 transmet au circuit de commande une information représentative de la position angulaire du boisseau 72.

L'utilisation d'une telle valve inspiratoire 54 présente de nombreux avantages.

Le fonctionnement de l'appareil d'aide à la ventilation 10, ainsi que la régulation du flux de gaz transmis aux voies aériennes supérieures du patient par la valve inspiratoire 52 sont les suivants.

En phase expiratoire, le boisseau 72 de la valve inspiratoire 52 est dans une position dite fermée, représentée à la figure 12. Dans ce cas, la jupe 74 obture totalement l'ouverture oblongue 66.

Avantageusement, dans cette position, le bord d'extrémité avant 80 de la jupe 74 n'obture pas totalement la première ouverture oblongue 66 de façon à laisser circuler en permanence, par la section de passage, un débit de gaz appelé débit de fuite.

Le débit de fuite qui circule dans la conduite inspiratoire 14 permet notamment de compenser les fuites au niveau du masque 15 du patient.

Lorsque l'appareil d'aide à la ventilation 10 passe en phase inspiratoire, le moteur 94 entraîne en rotation le boisseau 72 jusqu'à une position prédéterminée, représentée à la figure 13, de façon à augmenter la section de passage du gaz dans l'ouverture oblongue 66.

Lorsque la différence de pression entre l'amont et l'aval de la valve inspiratoire 52 est constante, le débit de gaz qui traverse la valve inspiratoire 52 est constant, et il est proportionnel à la section de passage du flux de gaz dans l'ouverture oblongue 66. Ainsi, le débit de gaz transmis aux voies aériennes supérieures du patient est déterminé avec précision.

Le passage de la position fermée à la position prédéterminée doit être le plus rapide possible. En effet c'est au début de la phase inspiratoire que le débit de gaz inspiré par le patient est le plus important. Lorsque le passage de la position fermée à la position prédéterminée n'est pas assez rapide, le patient doit faire un effort pour inspirer, ce qui provoque une gêne.

La rapidité du mouvement dépend de plusieurs paramètres, notamment de l'inertie des pièces en mouvement, des caractéristiques du moteur 94, ainsi que de l'angle de rotation à effectuer.

De façon à optimiser les performances de l'appareil d'aide à la ventilation 10, les pièces en mouvement sont réalisées dans des matériaux et selon des formes qui permettent de diminuer au maximum leur masse et leur inertie.

Le moteur 94 permet une grande accélération de façon à atteindre le plus rapidement possible sa vitesse maximale.

L'angle de rotation à effectuer par le boisseau 72 dépend du profil de son bord d'extrémité avant 80 ainsi que du type de ventilation requis par le patient.

Lorsque l'appareil d'aide à la ventilation 10 fonctionne en mode d'assistance respiratoire par ventilation volumétrique, il doit fournir au patient un volume de gaz prédéterminé.

Au cours de la phase inspiratoire, le débit de gaz transmis au patient peut être constant, certaines pathologies nécessitent une évolution du débit qui peut être par exemple croissante, décroissante ou sinusoïdale.

Pour ce type de ventilation, le circuit de commande de l'appareil 10 commande la pression du gaz à la sortie de la source de gaz de façon que la différence de pression entre l'amont et l'aval de la valve inspiratoire 52 soit maintenue constante. De plus, le circuit de commande de l'appareil 10 commande la rotation du boisseau 72 de façon que la section de passage de l'orifice oblongue 66 permette la transmission du volume de gaz prédéterminé.

Lorsque le débit doit être constant au cours de la phase inspiratoire, le moteur 94 positionne le boisseau 72 dans la position prédéterminée permettant la transmission du volume de gaz prédéterminé. L'angle de rotation boisseau 72, qui dépend du profilé du bord d'extrémité avant 80, est toujours inférieur à un tour.

Si le débit de gaz doit varier au cours de la phase inspiratoire, le moteur 94 positionne le boisseau 72 dans une position initiale, puis il provoque sa rotation au cours de la phase inspiratoire de façon que le volume de gaz transmis, au cours de la phase inspiratoire, corresponde au volume de gaz prédéterminé.

Lorsque l'appareil d'aide à la ventilation 10 fonctionne en mode d'assistance respiratoire par ventilation barométrique, il doit fournir au patient du gaz sous une pression prédéterminée.

Dans ce cas, le débit de gaz transmis au patient peut être élevé. De plus, c'est au début de la phase inspiratoire que l'appel de gaz, et donc le débit de gaz, par le patient est le plus important.

Il faut donc que la valve inspiratoire 52 permette rapidement le passage du débit maximal de gaz à travers l'ouverture oblongue 66.

Pour ce faire, le moteur 94 provoque la rotation du boisseau 72 de quelques degrés de façon que la rainure 84 soit en vis-à-vis de l'ouverture oblongue 66. Ainsi, une faible rotation du moteur 94 permet de passer de la position fermée de la valve inspiratoire 52, à la position pleine ouverture permettant la transmission du débit maximal.

Lors d'un tel type de ventilation, la source de gaz 12 est commandée par le circuit de commande, à partir de la valeur de la pression dans la conduite inspiratoire 14 fournie par les deuxièmes moyens de mesure 58.

Selon une variante, l'ouverture oblongue 66 est élargie à l'une de ses extrémités axiales de façon à augmenter le débit du gaz dans la section de passage.

Dans ce cas il est nécessaire d'augmenter légèrement l'angle de rotation du boisseau de façon à dégager complètement l'ouverture oblongue 66 élargie.

Avantageusement, l'élargissement est réalisé sur le tronçon de l'ouverture oblongue 66 qui n'est découvert par la jupe 74 que lorsque la valve inspiratoire 52 est en position de pleine ouverture. Ainsi, le débit maximum qui la traverse est augmenté en mode barométrique, tandis que, en mode volumétrique, la précision de régulation n'est pas diminuée.

Lorsque l'appareil d'aide à la ventilation 10 fonctionne en mode d'assistance respiratoire par ventilation à percussions, au cours de la phase inspiratoire duquel l'appareil doit fournir au patient un débit oscillant autour d'un débit de gaz prédéterminé, le circuit de commande de l'appareil 10 commande une oscillation de la position du boisseau 72 autour d'une position angulaire prédéterminée correspondant au débit prédéterminé. Le circuit de commande permet aussi la commande de la pression fournie par la source de gaz 12 de façon à assurer une différence de pression constante entre l'amont et l'aval de la valve inspiratoire 52.

Ainsi, l'appareil d'aide à la ventilation 10 selon l'invention permet de réguler de façon précise et rapide le flux de gaz sous pression transmis au patient.

Pour des raisons de sécurité, l'appareil d'aide à la ventilation 10 comporte aussi deux soupapes 99 de mise à l'air libre. Ainsi, si la source 12 de gaz sous pression ou la valve inspiratoire 52 venait à tomber en panne et à bloquer la circulation du flux d'air dans le bloc de distribution 50 lorsque le patient est en phase inspiratoire, les soupapes 99 de mise à l'air libre peuvent s'ouvrir lorsque la valeur de la dépression dans le deuxième trou 70 est inférieure à une valeur prédéterminée. Le patient peut ainsi aspirer de l'air extérieur. Dans ce cas, une valve expiratoire de l'appareil 10 est en position ouverte, de façon que le patient puisse expirer l'air qu'il a aspiré à travers les soupapes 99.

L'invention propose aussi que la source de gaz 12 sous pression comporte une machine électrique tournante 100, d'axe Y-Y, dont une extrémité libre du rotor 102 est fixée sur une roue à aubes 104.

La roue à aube 104 est montée rotative autour de l'axe Y-Y, dans une volute de guidage 106 d'un carter 108

Lorsque la machine électrique tournante 100 est alimentée électriquement, elle provoque la rotation de la roue à aubes 104 qui entraîne, dans la volute de guidage 106, un flux gazeux depuis une entrée axiale d'aspiration d'air 105 vers l'orifice de sortie 107. Le flux de gaz est transmis au patient. La source de gaz 12 fonctionne alors en mode "ventilateur".

Il est avantageux que le carter 108 comporte au moins un point 110, débouchant dans la volute 106, d'injection d'au moins un gaz qui est fourni par une source amont de gaz sous pression, non représentée, sous une seconde pression supérieure à la première pression du gaz transmis au patient qui est ici appelé gaz d'assistance.

Le gaz injecté dans la volute 106, au point d'injection 110, sous la seconde pression, peut alors permettre alors d'entraîner en rotation la roue à aubes 104, ainsi que le rotor 102 de la machine électrique tournante 100.

La machine électrique tournante 100 fonctionne alors en mode de génératrice d'énergie électrique.

Avantageusement, de façon que la machine électrique tournante produise le maximum d'énergie électrique, le flux d'au moins un gaz sous une seconde pression est injecté dans le carter 108 selon une direction sensiblement tangentielle à la volute 106.

Une telle source de gaz 12 permet alors d'augmenter l'autonomie de l'appareil d'aide à la ventilation 10. En effet, l'énergie électrique produite peut être utilisée par tous les organes consommateurs d'énergie électrique tels que le moteur 94 et le circuit de commande. De plus, l'énergie électrique produite peut permettre d'alimenter et de recharger les batteries d'accumulateurs électriques de l'appareil 10.

La source de pression amont consiste par exemple, au moins partiellement, en un circuit de gaz sous pression disponible en milieu hospitalier.

La source de pression amont peut aussi fournir à l'appareil un gaz thérapeutique tel que de l'oxygène.

Le dosage de la quantité de gaz thérapeutique injectée est avantageusement réalisé par une valve de dosage comportant un corps et un boisseau, de façon similaire à la valve inspiratoire 52. Ainsi, l'ouverture de cette valve de dosage du gaz thérapeutique peut être commandée de façon proportionnelle à l'ouverture de la valve inspiratoire 52.

Lorsque de l'air sous pression est fourni en permanence à l'appareil d'aide à la ventilation 10, l'énergie électrique produite par la machine électrique tournante 100 peut être suffisante au fonctionnement de l'appareil d'aide à la ventilation 10. Dans ce cas, l'alimentation électrique de l'appareil 10 par le secteur peut être supprimée. Cela permet de faciliter la manipulation de l'appareil 10 puisqu'il n'est pas relié au secteur par un fil électrique.

La source de pression amont peut aussi être un réservoir de gaz sous pression qui peut être disposé à proximité de l'appareil d'aide à la ventilation 10. La source de pression amont peut aussi être intégrée à l'appareil 10.

Avantageusement, des moyens de régulation de pression, non représentés, sont interposés entre la source de pression amont et le point d'injection 110. Ainsi, la source de pression amont peut fournir du gaz sous une pression supérieure à celle sous laquelle il doit être injecté dans le point d'injection 110, les moyens de régulation de la pression permettant d'adapter la pression du gaz injecté, de façon à permettre un rendement optimal de la machine électrique tournante 100.

Les moyens de régulation de pression peuvent être un détendeur de gaz.

De façon que l'appareil d'aide à la ventilation 10 puisse être utilisé par une grande diversité de patients, c'est à dire aussi bien par des patients ayant une certaine mobilité tels que des patients valides soignés à domicile que par des patients alités, notamment en milieu hospitalier, l'invention propose que l'appareil d'aide à la ventilation 10 soit de conception modulaire.

A cet effet appareil d'aide à la ventilation 10, est destiné notamment aux patients ayant une certaine mobilité et nécessitant un matériel simple, léger, maniable et robuste. L'appareil d'aide à la ventilation 10 qui comporte alors un circuit dit "circuit simple" 118, c'est-à-dire que la conduite inspiratoire 14 comporte à proximité du patient une valve expiratoire 120, conformément à la figure 13.

La valve expiratoire 120 est pilotée de façon à rester fermée lors de la phase inspiratoire et de s'ouvrir lors de la phase expiratoire.

Dans certains cas, lors de la phase expiratoire, il est nécessaire d'appliquer sur la valve expiratoire 120 une pression d'expiration positive permettant de compenser une pression intrinsèque aux poumons du patient.

Ainsi, le bloc de distribution comporte des moyens de pilotage 122 qui permettent d'une part d'ouvrir et de fermer la valve expiratoire 120 lors des phases expiratoire et inspiratoire du patient et, d'autre part, de piloter la valeur de la pression d'expiration positive (Pep).

Le pilotage de la valve expiratoire 120 peut être obtenu par l'application d'une pression sur une paroi mobile de la valve 120 permettant, ou non, l'évacuation vers l'extérieur du gaz expiré par le patient.

De façon connue, les moyens de pilotages comportent un compresseur, par exemple du type à palette(s) ou à membrane(s) pour piloter la valeur de la pression expiratoire positive.

Lors de son fonctionnement, le compresseur permet de fournir périodiquement un volume de gaz déterminé. Il est nécessaire d'agencer une chambre de stockage qui forme réserve de gaz, et permet d'augmenter et de stabiliser la pression du gaz fourni. Un orifice de sortie de la chambre est relié à la valve expiratoire 120, de façon notamment à appliquer une pression de pilotage sur sa paroi mobile.

De faibles variations de la pression de pilotage provoquent des oscillations de la paroi mobile de la valve 120, ainsi qu'une instabilité de la pression expiratoire positive. Cette instabilité peut provoquer une gène du patient.

Un tel type de compresseur ne permet pas un fonctionnement optimal de l'appareil 10 d'aide à la ventilation. À chaque fois qu'il fournit un volume de gaz déterminé, la chambre de stockage ne permet pas de stabiliser complètement la pression, ce qui provoque une variation de la pression expiratoire positive.

De plus, il est nécessaire de trouver un compromis entre le débit de la pompe et la stabilité de la pression expiratoire positive. En effet, un compresseur fournissant un débit des gaz important provoque des variations de la pression expiratoire positive qui sont plus importantes que celles provoquées par un compresseur fournissant un débit de gaz plus faible. Cependant, un débit de gaz important est nécessaire pour permettre un pilotage rapide de la valve 120 qui assure un confort optimal au patient.

De façon à remédier à ces inconvénients, l'invention propose que les moyens de pilotage 122 comportent un ventilateur constitué d'une machine électrique tournante, ainsi que d'une roue à aubes, de façon similaire à la source de gaz 12 décrite précédemment.

Ainsi, la machine électrique tournante du ventilateur est commandée par le circuit de commande de façon que la pression de sortie du gaz comprimé corresponde à la valeur de la pression d'expiration positive.

Un tel ventilateur présente une faible inertie ce qui permet de fournir rapidement un débit de gaz important sous une pression déterminée. Ainsi, le pilotage des moyens 120 est rapide.

De plus, la pression de sortie du ventilateur est sensiblement constante, ce qui permet de supprimer la chambre de stockage et d'assurer une stabilité optimale de la pression expiratoire positive.

De plus, le circuit de commande de l'appareil 10 commande la pression de sortie du ventilateur à partir de la pression du gaz expiré par le patient. Ainsi la pression expiratoire positive est indépendante du débit du gaz expiré par le patient, ainsi que de l'éventuelle valeur du débit de fuite de la source de gaz 12.

Un conduit 124 permet de relier les moyens de pilotage 122 à la valve expiratoire 120 de façon que le gaz comprimé applique un effort, sur une membrane mobile de la valve expiratoire 120, pour provoquer son ouverture lorsque la pression du gaz expiré par le patient est supérieure à la pression d'expiration positive.

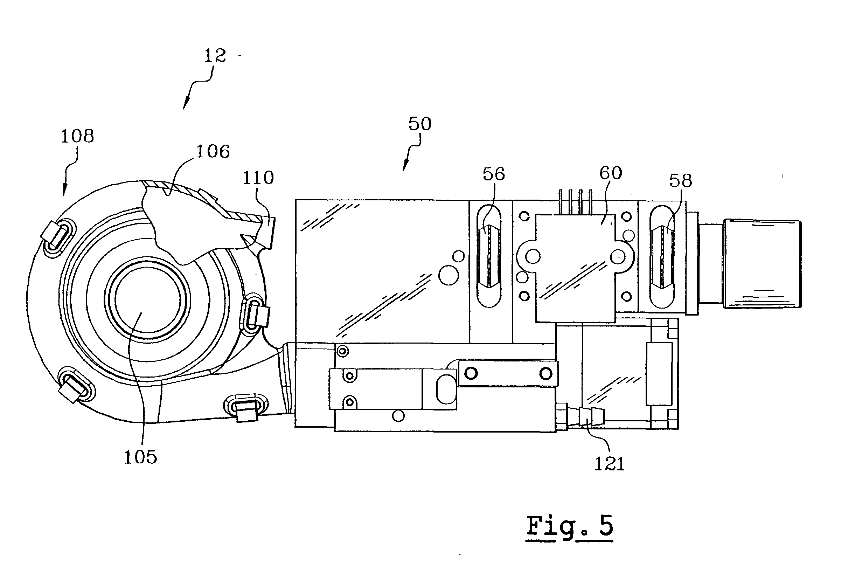

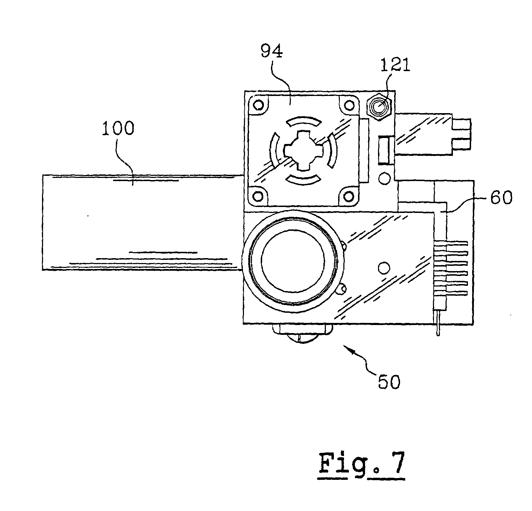

Pour faciliter le raccordement entre le conduit 124 et les moyens de pilotage 122, le bloc de distribution 50 comporte un connecteur 121, représenté notamment aux figures 5 et 7.

La pression et le débit du gaz comprimé circulant dans le conduit 124 sont très faibles. Ainsi, le conduit 124 peut être flexible et de petites dimensions.

De façon à contrôler l'air expiré par le patient, l'appareil d'aide à la ventilation 10 comporte des moyens de mesure 126 qui déterminent le débit du gaz qui circule dans la conduite inspiratoire, lors de la phase expiratoire. Les valeurs déterminées sont ensuite traitées par les moyens de commande 34 de façon à estimer le volume de gaz expiré par le patient.

Le bloc de distribution 50 intègre les moyens de mesure 126 qui consistent ici en deux capteurs de pression 125.

Ces deux capteurs 125 sont reliés à deux prises de pression 127 de la conduite inspiratoire 14 par l'intermédiaire de deux conduits 129.

Les prises de pression sont agencées de part et d'autre d'un organe 128 situé à proximité du masque 15 et qui a pour fonction de provoquer une chute de pression. Les moyens de commande 34 déterminent alors le volume expiré par le patient à partir de l'information représentative de la différence de pression fournie par les deux capteurs 125.

Ainsi, lorsque l'appareil 10 est équipé d'un circuit simple 118, la valve expiratoire 120 ainsi que les deux prises de pression 127 sont reliées aux moyens de pilotage 122 et aux moyens de mesure 126 respectivement par des conduits 124, 129 flexibles et de petites dimensions, de façon que le circuit simple 118 d'aspiration soit maniable, léger et peu encombrant ce qui permet une utilisation facile du masque 15.

De plus, le circuit simple 118 utilise des composants, tels que la valve expiratoire 120, robustes et peu coûteux.

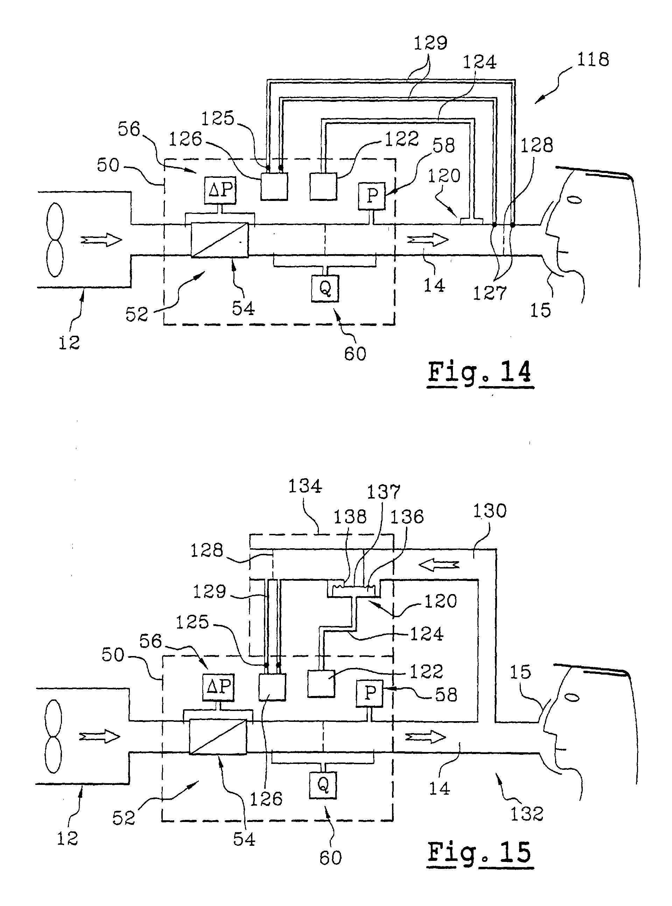

Le bloc de distribution 50 comportant les moyens de pilotage 122, ainsi que les moyens de mesure 126, peuvent aussi être utilisés pour un appareil d'aide à la ventilation 10 comportant un circuit dit "circuit double" 132 du type de celui représenté à la figure 15.

L'appareil d'aide à la ventilation 10 comporte alors une conduite inspiratoire 14 et une conduite expiratoire 130 qui sont reliées entre elles à proximité du masque 15.

Dans ce cas, l'extrémité aval de la conduite d'expiration 130 comporte un module 134 qui est fixé sur le bloc de distribution 50.

Le module 134 comporte l'organe dépressionnaire 128 ainsi qu'une valve expiratoire 120 qui sont reliés aux moyens de mesure 126 et aux moyens de pilotage 122 respectivement par les conduits 124, 129 réalisés dans un module 134.

Des éléments d'étanchéité sont interposés autour des extrémités libres des conduits 124, 129 de façon à éviter toute fuite qui perturberait la mesure du volume expiré ou la commande de la pression d'expiration positive de la valve expiratoire 120.

La longueur des conduits 124, 129 étant très faible, la valeur de la pression d'expiration positive et la mesure du volume expiré sont très précises.

L'appareil d'aide à la ventilation 10 équipé d'un circuit double 132 peut être destiné à des patients dont l'état de santé est critique. Il est alors nécessaire de supprimer toutes les perturbations pouvant diminuer ses performances.

On recense deux causes principales de la diminution des performances qui sont la condensation dans la conduite d'expiration 130 et la résistance à l'ouverture de la valve expiratoire 120.

En effet, la condensation dans la conduite d'expiration 130, et notamment dans le tronçon situé dans le module 134, peut perturber la mesure du volume expiré, ainsi que le fonctionnement de la valve expiratoire 120.

De façon à minimiser, voir à supprimer, la condensation, notamment à l'extrémité aval de la conduite d'expiration 130, le module 134 est chauffé pour provoquer l'évaporation des particules d'eau formées.

De plus, le tronçon d'extrémité aval de la conduite d'expiration 130 est globalement incliné de façon que son extrémité libre aval soit au niveau le plus bas de la conduite 130, de façon à faciliter l'évacuation des condensats.

En général, la valve expiratoire 120 des appareils 10 d'aide à la ventilation équipés d'un circuit double 132 est de la forme "valve ballon" dans laquelle une pression d'air à l'intérieur d'un ballon 136 force une paroi mobile 137 de celui-ci à se plaquer contre un siège annulaire 138 pour obturer un orifice d'entrée de la valve. Lorsque la pression à l'entrée de la valve 120 est supérieure à la pression à l'intérieur du ballon 136, la valve 120 est alors susceptible de s'ouvrir, en permettant ainsi le passage du gaz au travers de la valve 120, généralement vers l'atmosphère.

Un tel type de valve permet une bonne maîtrise de la pression d'expiration positive lorsque l'on est dans un régime établi. Cependant, lors de la phase d'ouverture de la valve 120, la pression qui doit régner dans la conduite expiratoire 130 doit être supérieure à la pression expiratoire positive. La différence entre ces deux pressions provient de la déformation élastique du ballon 136.

Or c'est en général au début de la phase expiratoire que le débit expiré par le patient est le plus important. La surpression provoquée par la déformation élastique du ballon peut alors entraîner une gêne pour le patient.

Ainsi, il est avantageux d'équiper les moyens de pilotage 122 de la pression d'expiration positive d'un système d'aide à l'ouverture de la valve expiratoire 120. Un tel système permet, lors de la phase d'ouverture de la valve expiratoire 120, d'appliquer une pression qui correspond à la différence entre la pression d'expiration positive et la pression à appliquer pour provoquer l'ouverture de la valve expiratoire 120.

Le système d'aide à l'ouverture de la vanne expiratoire 120 peut aussi comporter un actionneur électromagnétique dont un élément mobile est relié à la paroi mobile 137. Ainsi, lors de l'ouverture de la valve expiratoire 120, l'élément mobile de l'actionneur électromagnétique applique sur la paroi mobile 137 un effort équivalent à l'effort résistant dû à la déformation élastique du ballon 136.

Un tel système d'aide à l'ouverture la vanne expiratoire 120 permet de combiner la rapidité de l'actionneur électromagnétique, lors de l'ouverture de la valve expiratoire 120, et la stabilité du pilotage de la pression expiratoire positive par les moyens 122 lorsque la valve expiratoire 120 est ouverte.

Lorsque la pression à appliquer pour provoquer l'ouverture de la valve expiratoire 120 est supérieure à la pression d'expiration positive, les moyens de pilotage 122 doivent appliquer une dépression dans le ballon 136.

L'appareil d'aide à la ventilation 10 peut ainsi être utilisé indifféremment avec un circuit simple 118 ou un circuit double 132. L'appareil 10 peut alors être utilisé indifféremment en milieu hospitalier ou à domicile.

De façon à faciliter l'adaptation du circuit simple 118 et du circuit double 132 sur le bloc de distribution 50, l'appareil d'aide à la ventilation 10 comporte des connecteurs, permettant de raccorder indifféremment les moyens de pilotage 122 et les moyens de mesure 126 et aux conduits 124, 129 respectivement d'un circuit simple 118 ou d'un circuit double 132.

Le bloc de distribution 50 comporte aussi des moyens de fixation, non représentés, du module 134.

Les moyens de fixation peuvent être des taraudages dans lesquels sont vissés des éléments filetés. Ils peuvent aussi comporter des éléments qui coopèrent de façon élastique avec des éléments complémentaires du module 134, de façon que ce dernier puisse être fixé au bloc de distribution 50 par engagement des éléments complémentaires.

Selon une variante, non représentée, la valve inspiratoire permet d'évacuer vers l'atmosphère au moins une partie du gaz fourni par la source de gaz 12, lors de certaines phases du cycle d'aide à la ventilation de l'appareil 10, et notamment lors de la phase expiratoire. Le document FR-A-2.714.837 décrit et représente une valve de ce type.

L'invention propose que la paroi cylindrique annulaire du corps tubulaire de la valve inspiratoire comporte une seconde ouverture ou lumière qui est d'orientation sensiblement perpendiculaire à la première ouverture. La seconde ouverture permet alors de relier l'intérieur du corps tubulaire et l'extérieur de l'appareil.

Le profil du bord d'extrémité axiale avant de la jupe à alors une forme générale en V.

Selon cette variante, la source de gaz fournit un flux de gaz constant, la régulation du flux transmis au patient étant alors réalisée par la position angulaire de la jupe dans le corps.

La première et la seconde ouvertures sont agencées l'une par rapport à l'autre de façon que la jupe puisse les obturer, ou non, de manière totale ou partielle.

En effet, par exemple en mode d'assistance respiratoire par ventilation barométrique, lors de la phase inspiratoire, la jupe obture totalement la seconde ouverture, de façon que l'intégralité du flux de gaz fourni par la source 12 soit transmise à la conduite principale inspiratoire.

Lors de la phase expiratoire, la jupe obture totalement (ou partiellement pour permettre le passage du débit de fuite) la première ouverture.

De façon à éviter une augmentation de la pression à la sortie de la source de gaz, la position angulaire de la jupe permet l'ouverture de la deuxième ouverture pour évacuer le gaz vers l'atmosphère.

Avantageusement, la forme et la position de la première et de la seconde ouvertures, ainsi que de la jupe du boisseau sont telles que la section de passage cumulée du gaz à travers la première et la seconde ouverture est constante, quelle que soit la phase de fonctionnement de l'appareil d'aide à la ventilation.

## Revendications

1. Appareil d'aide à la ventilation (10) d'un patient qui respire par des cycles successifs dont chacun comporte une phase inspiratoire et une phase expiratoire, du type qui comporte :
- une source de gaz (12) sous pression dont un orifice de sortie fournit un flux de gaz sous pression destiné à être transmis aux voies aériennes supérieures du patient ;
- un bloc de distribution (50) du flux de gaz sous pression qui comprend un circuit de transmission (52) qui relie l'orifice de sortie (107) de la source de gaz (12) à une première extrémité libre (54) d'une conduite principale inspiratoire (14) sur la seconde extrémité libre de laquelle est fixé un masque (15), notamment du type facial, destiné à être porté par le patient ; et
- une valve inspiratoire (54) de régulation dudit flux de gaz qui est interposée dans le circuit de transmission (52), qui est commandée par un circuit de commande de l'appareil 10, en fonction notamment des valeurs du débit et de la pression du gaz dans la conduite principale (14), et qui est réalisée sous la forme d'un distributeur rotatif,
**caractérisé en ce que** la valve inspiratoire (54) de régulation du flux de gaz comporte un corps (62) de valve tubulaire comportant, dans sa paroi, une ouverture oblongue longitudinale (66) qui permet la transmission du flux de gaz depuis l'orifice de sortie (107) de la source de gaz (12) à la première extrémité libre de la conduite principale inspiratoire (14) et comporte, un boisseau (72) qui est monté mobile en rotation dans le corps (62), qui est fermé à l'une de ses extrémités axiales et dont un bord d'extrémité axiale (80) opposé à son extrémité fermée comporte un profil en forme de spirale (86) de façon à provoquer, lors de la rotation du boisseau (72), la variation progressive de la section de passage de l'ouverture oblongue (66) permettant la transmission du flux de gaz.

2. Appareil d'aide à la ventilation (10) selon la revendication précédente, **caractérisé en ce que** l'orifice de sortie (107) de la source de gaz (12) débouche dans la première extrémité libre d'un premier trou (64) dont la seconde extrémité libre est obturée par l'extrémité fermée du boisseau (72), et **en ce que** le bloc de distribution (50) comporte un évidement (68) qui débouche d'une part en vis-à-vis de l'ouverture oblongue longitudinale (66) de laquelle il est complémentaire et, d'autre part, dans un deuxième trou (70) borgne sur l'extrémité libre duquel est fixée la première extrémité libre de la conduite principale inspiratoire (14).

3. Appareil d'aide à la ventilation (10) selon l'une des revendications 1 ou 2, **caractérisé en ce que** le boisseau (72) est entraîné en rotation par un moteur (94), notamment du type pas à pas, commandé par le circuit de commande de l'appareil (10).

4. Appareil d'aide à la ventilation (10) selon l'une quelconque des revendications précédentes, **caractérisé en ce que** le boisseau (72) comporte un dispositif d'indexation (96) de sa position angulaire.

5. Appareil d'aide à la ventilation (10) selon l'une quelconque des revendications précédentes, **caractérisé en ce que** le circuit de commande de l'appareil (10) commande la position angulaire du boisseau (72) et la pression fournie par la source de gaz (12) de façon que la section de passage permette la transmission au patient du volume de gaz prédéterminé.

6. Appareil d'aide à la ventilation (10) selon l'une quelconque des revendications précédentes, **caractérisé en ce que** le circuit de commande de l'appareil (10) commande la position angulaire du boisseau (72) de façon que la section de passage corresponde à la totalité de l'ouverture oblongue (66) et, **en ce que** le circuit de commande de l'appareil (10) commande la pression fournie par la source de gaz (12), pour que la pression du gaz dans la conduite inspiratoire (14) soit la pression de gaz prédéterminée.

7. Appareil d'aide à la ventilation (10) selon la revendication précédente, **caractérisé que** le circuit de commande de l'appareil (10) commande l'oscillation de la position angulaire du boisseau (72) autour d'une position prédéterminée correspondant au débit prédéterminé et commande la pression fournie par la source de gaz (12).

8. Appareil d'aide à la ventilation (10) selon l'une quelconque des revendications précédentes, **caractérisé en ce que** la source de gaz (12) sous pression comporte une machine électrique tournante (100), pilotée en vitesse par le circuit de commande, dont une extrémité libre du rotor (102) entraîne en rotation une roue à aubes (104) qui entraîne le flux gazeux dans une volute de guidage (106) d'un carter (108), lorsque la machine électrique tournante (100) est alimentée électriquement.

9. Appareil d'aide à la ventilation (10) selon la revendication précédente, **caractérisé en ce que** le carter (108) comporte au moins un point d'injection (110), débouchant dans la volute (106), d'au moins un gaz comprimé qui est fourni par une autre source de pression amont, sous une seconde pression supérieure à la première pression, et **en ce que** le gaz comprimé sous la seconde pression est susceptible d'entraîner en rotation la roue à aubes (104) ainsi que le rotor de la machine électrique tournante (100), de façon que le gaz sous pression soit fourni au patient sous la première pression et que la machine électrique tournante (100) fonctionne en génératrice d'énergie électrique et produise de l'énergie électrique.

10. Appareil d'aide à la ventilation (10) selon la revendication précédente, **caractérisé en ce que** le flux d'au moins un gaz sous une seconde pression est injecté dans le carter (108) selon une direction sensiblement tangentielle à la volute (106).

11. Appareil d'aide à la ventilation (10) selon l'une des revendications 9 ou 10, **caractérisé que** la source de pression amont consiste au moins partiellement en un circuit de gaz sous pression, disponible en milieu hospitalier.

12. Appareil d'aide à la ventilation (10) selon l'une quelconque des revendications 9 à 11, **caractérisé en ce que** la source de pression comporte un réservoir de gaz sous pression.

13. Appareil d'aide à la ventilation (10) respiratoire selon la revendication précédente, **caractérisé en ce que** le réservoir est intégré à l'appareil d'aide à la ventilation (10).

14. Appareil d'aide à la ventilation (10) respiratoire selon l'une quelconque des revendications 9 à 13, **caractérisé en ce que** le gaz sous la seconde pression est composé au moins partiellement d'air.

15. Appareil d'aide à la ventilation (10) respiratoire selon l'une quelconque des revendications 9 à 14, **caractérisé en ce que** le gaz comprimé sous la seconde pression est composé au moins partiellement d'un gaz thérapeutique.

16. Appareil d'aide à la ventilation (10) selon la revendication précédente, **caractérisé en ce que** le gaz thérapeutique est de l'oxygène.

17. Appareil d'aide à la ventilation (10) selon l'une quelconque des revendications 9 à 16, **caractérisé en ce que** des moyens de régulation de pression sont interposés entre ladite autre source de pression amont et le point d'injection (110).

18. Appareil d'aide à la ventilation (10) selon l'une quelconque des revendications 9 à 17, **caractérisé en ce qu'**il comporte une valve de dosage du gaz comprimé sous la seconde pression, dont l'ouverture est commandée de façon proportionnelle à l'ouverture de la valve inspiratoire (54).

19. Appareil d'aide à la ventilation (10) respiratoire selon l'une quelconque des revendications 8 à 18, **caractérisé en ce que** l'énergie électrique fournie par la machine électrique tournante (100), fonctionnant en génératrice, alimente au moins partiellement les systèmes consommateurs d'électricité de l'appareil (10).

20. Appareil d'aide à la ventilation (10) respiratoire selon l'une quelconque des revendications 9 à 19, **caractérisé en ce que** l'énergie électrique produite par la machine électrique tournante (100), fonctionnant en génératrice, alimente et charge au moins partiellement une batterie d'accumulateurs de l'appareil (10).

21. Appareil d'aide à la ventilation (10) respiratoire selon l'une quelconque des revendications 9 à 20, **caractérisé en ce que** l'énergie électrique produite par la machine électrique tournante (100) est supérieure à l'énergie électrique consommée par les systèmes consommateurs d'électricité, de façon que l'appareil d'aide à la ventilation (10) respiratoire soit autonome.

22. Appareil d'aide à la ventilation (10) respiratoire selon l'une quelconque des revendications précédentes, **caractérisé en ce que** le bloc de distribution (50) comporte des moyens de pilotage (122) qui permettent de piloter l'ouverture et la fermeture d'une valve expiratoire (120) qui est agencée indifféremment dans la conduite inspiratoire (14) d'un circuit simple (118) ou dans la conduite expiratoire (130) d'un circuit double (132).

23. Appareil d'aide à la ventilation (10) selon la revendication précédente, **caractérisé en ce que**, lorsque qu'il comporte un circuit double (132), la vanne expiratoire (120) est agencée dans un module (134) qui est fixé sur le bloc de distribution (50) et qui comporte un conduit (124) de façon à relier les moyens de pilotage (122) à la vanne expiratoire (120).

24. Appareil d'aide à la ventilation (10) selon l'une des revendications 22 ou 23, **caractérisé en ce que** les moyens de pilotage (122) permettent d'appliquer à la valve expiratoire (120) une pression expiratoire positive, et **en ce que** la pression expiratoire positive est fournie par un ventilateur.

25. Appareil d'aide à la ventilation (10) selon l'une quelconque des revendications précédentes, **caractérisé en ce que** le bloc de distribution (50) comporte des moyens de mesure (126) qui déterminent le débit du gaz qui circule, lors de la phase expiratoire, dans la conduite inspiratoire (14) d'un circuit simple (118), ou dans la conduite expiratoire (130) d'un circuit double (132).

26. Appareil d'aide à la ventilation (10) selon la revendication précédente, **caractérisé en ce que**, lorsque qu'il comporte un circuit double (132), il comprend un module (134) qui est fixé sur le bloc de distribution (50) et qui comporte au moins un conduit (129) qui relie les moyens de mesure (126) à la conduite expiratoire (130).

## Claims

1. Breathing assistance apparatus for a patient breathing in successive cycles, each of which comprises a phase of inhalation and a phase of exhalation, of the type comprising:
- a pressurized gas source (12) with an outlet orifice suppling a stream of pressurized gas intended to be transmitted to the upper airways of the patient;
- a pressurized gas stream distribution unit (50) comprising a transmission circuit (52) which connects the outlet orifice (107) of the gas source (12) to a first free end (54) of a main inhalation duct (14), to the second free end of which is fixed a mask (15), particularly of the face mask type, intended to be worn by the patient; and
- an inhalation valve (54) for regulating the said gas stream and which is interposed in the transmission circuit (52), and is controlled by a control circuit of the apparatus (10) as a function in particular of the values of the flow rate and of the pressure of the gas in the main duct (14), and which has the form of a rotary directional control valve,
**characterized in that** the inhalation valve (54) for regulating the gas stream comprises a tubular valve body (62) comprising, in its wall, a longitudinal oblong opening (66) which allows the stream of gas from the outlet orifice (107) of the gas source (12) to be transmitted to the first free end of the main inhalation duct (14) and comprises a barrel (72) which is mounted so that it can turn in the body (62), which is closed at one of its axial ends, and of which an axial end edge (80), at the opposite end to its closed end, comprises a spiral-shaped profile (86) so that, as the barrel (72) is turned, the passage cross section of the oblong opening (66) which allows the transmission of the gas stream is varied progressively.

2. Apparatus (10) for assisting with ventilation according to the preceding claim, **characterized in that** the outlet orifice (107) of the gas source (12) opens into the first free end of a first hole (64), the second free end of which is plugged by the closed end of the barrel (72) and **in that** the distribution unit (50) has a recess (68) which opens, on the one hand, opposite the longitudinal oblong opening (66) which it complements, and on the other hand, into a second blind hole (70) to the free end of which the first free end of the main inhalation duct (14) is fixed.

3. Apparatus (10) for assisting with ventilation according to either of Claims 1 and 2, **characterized in that** the barrel (72) is turned by a motor (94), particularly of the stepping type, controlled by the control circuit of the apparatus (10).

4. Apparatus (10) for assisting with ventilation according to any one of the preceding claims, **characterized in that** the barrel (72) has a device (96) for indexing its angular position.

5. Apparatus (10) for assisting with ventilation according to any one of the preceding claims, **characterized in that** the control circuit of the apparatus (10) controls the angular position of the barrel (72) and the pressure supplied by the gas source (12) so that the passage cross section allows the predetermined volume of gas to be transmitted to the patient.

6. Apparatus (10) for assisting with ventilation according to any one of the preceding claims, **characterized in that** the control circuit of the apparatus (10) controls the angular position of the barrel (72) so that the passage cross section corresponds to the entirety of the oblong opening (66) and **in that** the control circuit of the apparatus (10) controls the pressure supplied by the gas source (12) so that the pressure of the gas in the inhalation duct (14) is the predetermined gas pressure.

7. Apparatus (10) for assisting with ventilation according to the preceding claim, **characterized in that** the control circuit of the apparatus (10) controls the switching of the angular position of the barrel (72) back and forth about a predetermined position that corresponds to the predetermined flow rate and controls the pressure supplied by the gas source (12).

8. Apparatus (10) for assisting with ventilation according to any one of the preceding claims, **characterized in that** the pressurized gas source (12) comprises a rotary electric machine (100) controlled in terms of speed by the control circuit, a free end of the rotor (102) of which drives the rotation of a bladed wheel (104) which drives the gas stream through a guide volute (106) of a casing (108) when the rotary electric machine (100) is electrically powered.

9. Apparatus (10) for assisting with ventilation according to the preceding claim, **characterized in that** the casing (108) has at least one injection point (110), opening into the volute (106), for ejecting at least one compressed gas which is supplied by another upstream pressure source, at a second pressure higher than the first pressure, and **in that** the compressed gas at the second pressure is capable of driving the rotation of the bladed wheel (104) and of the rotor of the rotary electric machine (100) so that the pressurized gas is supplied to the patient at the first pressure and the rotary electric machine (100) acts as an electricity generator and produces electrical energy.

10. Apparatus (10) for assisting with ventilation according to the preceding claim, **characterized in that** the stream of at least one gas at a second pressure is injected into the casing (108) in a direction more or less tangential to the volute (106).

11. Apparatus (10) for assisting with ventilation according to either of Claims 9 and 10, **characterized in that** the upstream pressure source at least partially consists of a pressurized gas circuit available in a hospital environment.

12. Apparatus (10) for assisting with ventilation according to any one of Claims 9 to 11,
**characterized in that** the source of pressure comprises a pressurized gas reservoir.

13. Apparatus (10) for assisting with respiratory ventilation according to the preceding claim, **characterized in that** the reservoir is incorporated into the apparatus (10) for assisting with ventilation.

14. Apparatus (10) for assisting with respiratory ventilation according to any one of Claims 9 to 13, **characterized in that** the gas at the second pressure is made up at least partially of air.

15. Apparatus (10) for assisting with respiratory ventilation according to any one of Claims 9 to 14, **characterized in that** the compressed gas at the second pressure is made up at least partially of a therapeutic gas.

16. Apparatus (10) for assisting with ventilation according to the preceding claim, **characterized in that** the therapeutic gas is oxygen.

17. Apparatus (10) for assisting with ventilation according to any one of Claims 9 to 16, **characterized in that** means for regulating the pressure are inserted between the said other upstream pressure source and injection point (110).

18. Apparatus (10) for assisting with ventilation according to any one of Claims 9 to 17, **characterized in that** it comprises a valve for metering the compressed gas at the second pressure, the opening of which valve is controlled in proportion with the opening of the inhalation valve (54).

19. Apparatus (10) for assisting with respiratory ventilation according to any one of Claims 8 to 18, **characterized in that** the electrical energy supplied by the rotary electric machine (100), operating as a generator, at least partially powers the electricity-consuming systems of the apparatus (10).

20. Apparatus (10) for assisting with respiratory ventilation according to any one of Claims 9 to 19, **characterized in that** the electrical energy produced by the rotary electric machine (100), operating as a generator, powers and at least partially charges an accumulator battery of the apparatus (10).

21. Apparatus (10) for assisting with respiratory ventilation according to any one of Claims 9 to 20, **characterized in that** the electrical energy produced by the rotary electric machine (100) is more than the electrical energy consumed by the electricity-consuming systems, so that the apparatus (10) for assisting with respiratory ventilation is autonomous.

22. Apparatus (10) for assisting with respiratory ventilation according to any one of the preceding claims, **characterized in that** the distribution unit (50) comprises controlling means (122) which can order the opening and closing of an exhalation valve (120) which is arranged with equal preference in the inhalation duct (14) of a single circuit (118) or in the exhalation duct (130) of a double circuit (132).

23. Apparatus (10) for assisting with ventilation according to the preceding claim, **characterized in that**, when it has a double circuit (132), the exhalation valve (120) is arranged in the module (134) which is fixed to the distribution unit (50) and comprises a duct (124) for connecting the controlling means (122) to the exhalation valve (120).

24. Apparatus (10) for assisting with ventilation according to either of Claims 22 and 23, **characterized in that** the controlling means (122) allow a positive exhalation pressure to be applied to the exhalation valve (120), and **in that** the positive exhalation pressure is provided by a fan.

25. Apparatus (10) for assisting with ventilation according to any one of the preceding claims, **characterized in that** the distribution unit (50) comprises measuring means (126) which determine the flow rate of gas flowing, during the exhalation phase, through the inhalation duct (14) of a single circuit (118) or through the exhalation duct (130) of a double circuit (132).

26. Apparatus (10) for assisting with ventilation according to the preceding claim, **characterized in that**, when it has a double circuit (132) it comprises a module (134) which is fixed to the distribution unit (50) and comprises at least one duct (129) connecting the measuring means (126) to the exhalation duct (130).

## Patentansprüche

1. Vorrichtung zur Unterstützung bei der Beatmung (10) eines Patienten, der in aufeinander folgenden Zyklen atmet, von denen jeder eine Einatmungsphase und eine Ausatmungsphase aufweist, die umfasst:
eine Druckgasquelle (12), an deren einer Ausgangsöffnung ein Druckgas ausströmt, um in die oberen Atemwege des Patienten zu gelangen,
einen Verteilerblock (50) für das Gas unter Druck, der einen Übertragungskreis (52) umfasst, der die Ausgangsöffnung (107) der Gasquelle (12) mit einem ersten freien Ende (54) einer Haupteinatmungsleitung (14) verbindet, an deren zweitem freien Ende sich eine Maske (15) befindet, insbesondere eine Gesichtsmaske, die von dem Patienten getragen wird, und
ein Einatmungsventil (54) zum Regeln des Gasflusses, das in den Übertragungskreis (52), der durch einen Steuerkreis der Vorrichtung (10) gesteuert wird, insbesondere in Abhängigkeit von Durchsatzraten und dem Druck des Gases in der Hauptleitung (14), geschaltet ist und die Form eines Drehverteilers aufweist,
**dadurch gekennzeichnet, dass** das Einatmungsventil (54) für die Regelung des Gasflusses einen röhrenförmigen Ventilkörper (62) mit einer verlängerten Längsöffnung (66) in seiner Wand aufweist, die die Weiterleitung des Gases aus der Ausgangsöffnung (107) der Gasquelle (12) zu dem ersten freien Ende der Haupteinatmungsleitung (14) ermöglicht und einen Rollenkörper (72) umfasst, der in dem Körper (62) drehbar angeordnet ist, dessen axiale Enden geschlossen sind und dessen einer axialer Rand gegenüber seinem geschlossenen Ende ein Profil in Form einer Spirale (86) aufweist, so dass bei Drehung des Rollenkörpers (72) die progressive Veränderung des Querschnitts des Durchlasses der verlängerten Öffnung (66) die Übertragung des Gases ermöglicht.

2. Vorrichtung zur Unterstützung bei der Beatmung (10) nach dem vorangehenden Anspruch, **dadurch gekennzeichnet, dass** die Ausgangsöffnung (107) der Gasquelle (12) in dem ersten freien Ende eines ersten Loches (64) mündet, dessen zweites freie Ende durch das geschlossene Ende des Rollenkörpers (72) verschlossen wird, und dass der Verteilerblock (50) eine Aussparung (68) umfasst, die einerseits gegenüber der verlängerten Längsöffnung (66) mündet, zu der sie komplementär ist, und andererseits in einem zweiten Blindloch (70) mündet, an dessen freiem Ende das erste freie Ende der Haupteinatmungsleitung (14) befestigt ist.

3. Vorrichtung zur Unterstützung bei der Beatmung (10) nach einem der Ansprüche 1 oder 2, **dadurch gekennzeichnet, dass** der Rollenkörper (72) durch einen Motor (94) in Drehung versetzt wird, insbesondere einem Schrittmotor, der durch den Steuerschaltkreis der Vorrichtung (10) gesteuert wird.

4. Vorrichtung zur Unterstützung bei der Beatmung (10) nach einem der vorangehenden Ansprüche, **dadurch gekennzeichnet, dass** der Rollenkörper (72) eine Indexvorrichtung (96) für seine Winkelposition aufweist.

5. Vorrichtung zur Unterstützung bei der Beatmung (10) nach einem der vorangehenden Ansprüche, **dadurch gekennzeichnet, dass** der Steuerschaltkreis der Vorrichtung (10) die Winkelposition des Rollenkörpers (72) und den Druck von der Gasquelle (12) so einstellt, dass der Querschnitt des Durchlasses die Übertragung eines vorgegebenen Gasvolumens zum Patienten ermöglicht.

6. Vorrichtung zur Unterstützung bei der Beatmung (10) nach einem der vorangehenden Ansprüche, **dadurch gekennzeichnet, dass** der Steuerschaltkreis der Vorrichtung (10) die Winkelposition des Rollenkörpers (72) einstellt, so dass der Querschnitt des Durchlasses dem Gesamtmaß der verlängerten Öffnung (66) entspricht, und dass der Steuerschaltkreis der Vorrichtung (10) den Druck der Gasquelle (12) derart einstellt, dass der Gasdruck in der Einatmungsleitung (14) dem vorgegebenen Gasdruck entspricht.

7. Vorrichtung zur Unterstützung bei der Beatmung (10) nach dem vorangehenden Anspruch, **dadurch gekennzeichnet, dass** der Steuerschaltkreis der Vorrichtung (10) die Oszillation der Winkelposition des Rollenkörpers (72) um eine vorgegebene Position in Übereinstimmung mit dem vorgegebenen Durchsatz einstellt und den Druck der Gasquelle (12) steuert.

8. Vorrichtung zur Unterstützung bei der Beatmung (10) nach einem der vorangehenden Ansprüche, **dadurch gekennzeichnet, dass** die Druckgasquelle (12) eine elektrische Rotationsmaschine (100) umfasst, die in ihrer Geschwindigkeit durch den Steuerschaltkreis gesteuert wird, wobei ein freies Ende der Rotationsmaschine (102) ein Verdichterrad (104) antreibt, durch das das Gas in eine Führungsspirale (106) eines Gehäuses (108) geleitet wird, wenn die elektrische Rotationsmaschine (100) elektrisch versorgt wird.

9. Vorrichtung zur Unterstützung bei der Beatmung (10) nach dem vorangehenden Anspruch, **dadurch gekennzeichnet, dass** das Gehäuse (108) wenigstens einen Punkt (110) aufweist, der in der Spirale (106) mündet, um wenigstens ein komprimiertes Gas, das durch eine andere Druckquelle oberhalb bereitgestellt wird, bei einem zweiten Druck, der über dem ersten Druck liegt, zu injizieren, und dass das komprimierte Gas mit dem zweiten Druck dazu geeignet ist, das Verdichterrad (104) wie auch den Rotor der elektrischen Rotationsmaschine (100) in Drehung zu versetzen, so dass das Druckgas dem Patienten unter dem ersten Druck zugeführt wird und dass die elektrische Rotationsmaschine (100) als Generator für elektrische Energie arbeitet und elektrische Energie erzeugt.

10. Vorrichtung zur Unterstützung bei der Beatmung (10) nach dem vorangehenden Anspruch, **dadurch gekennzeichnet, dass** der Fluss des wenigstens einen Gases unter dem zweiten Druck in das Gehäuse (108) in einer im Wesentlichen tangential verlaufenden Richtung zur Spirale (106) injiziert wird.

11. Vorrichtung zur Unterstützung bei der Beatmung (10) nach einem der Ansprüche 9 und 10, **dadurch gekennzeichnet, dass** die Druckquelle oberhalb wenigstens teilweise aus einem Druckgaskreis besteht, der in einem Krankenhaus zur Verfügung steht.

12. Vorrichtung zur Unterstützung bei der Beatmung (10) nach einem der Ansprüche 9 bis 11, **dadurch gekennzeichnet, dass** die Druckquelle ein Druckgasreservoir umfasst.

13. Vorrichtung zur Unterstützung bei der respiratorischen Beatmung (10) nach dem vorangehenden Anspruch, **dadurch gekennzeichnet, dass** das Reservoir in der Vorrichtung zur Unterstützung bei der Beatmung (10) integriert ist.

14. Vorrichtung zur Unterstützung bei der respiratorischen Beatmung (10) nach einem der Ansprüche 9 bis 13, **dadurch gekennzeichnet, dass** das Gas unter dem zweiten Druck wenigstens teilweise Luft ist.

15. Vorrichtung zur Unterstützung bei der respiratorischen Beatmung (10) nach einem der Ansprüche 9 bis 14, **dadurch gekennzeichnet, dass** das komprimierte Gas unter dem zweiten Druck wenigstens teilweise aus einem therapeutischen Gas besteht.

16. Vorrichtung zur Unterstützung bei der Beatmung (10) nach dem vorangehenden Anspruch, **dadurch gekennzeichnet, dass** das therapeutische Gas Sauerstoff ist.

17. Vorrichtung zur Unterstützung bei der Beatmung (10) nach einem der Ansprüche 9 bis 16, **dadurch gekennzeichnet, dass** die Einrichtungen zum Einstellen des Druckes zwischen der anderen oberhalb liegenden Druckquelle und dem Injektionspunkt (110) angeordnet sind.

18. Vorrichtung zur Unterstützung bei der Beatmung (10) nach einem der Ansprüche 9 bis 17, **dadurch gekennzeichnet, dass** sie ein Dosierungsventil für das komprimierte Gas unter dem zweiten Druck umfasst, dessen Öffnung proportional zur Öffnung des Einatmungsventils (54) gesteuert wird.

19. Vorrichtung zur Unterstützung bei der respiratorischen Beatmung (10) nach einem der Ansprüche 8 bis 18, **dadurch gekennzeichnet, dass** die elektrische Energie von der elektrischen Rotationsmaschine (100), die als Generator arbeitet, wenigstens teilweise die elektrischen Verbraucher der Vorrichtung (10) versorgt.

20. Vorrichtung zur Unterstützung bei der respiratorischen Beatmung (10) nach einem der Ansprüche 9 bis 19, **dadurch gekennzeichnet, dass** die elektrische Energie von der elektrischen Rotationsmaschine (100), die als Generator arbeitet, für die Versorgung dient und wenigstens teilweise eine Akkumulatorbatterie der Vorrichtung (10) lädt.

21. Vorrichtung zur Unterstützung bei der respiratorischen Beatmung (10) nach einem der Ansprüche 9 bis 20, **dadurch gekennzeichnet, dass** die elektrische Energie von der elektrischen Rotationsmaschine (100) größer als die elektrische Energie ist, die durch die elektrischen Verbraucher verbraucht wird, so dass die Vorrichtung zur Unterstützung bei der respiratorischen Beatmung (10) autonom ist.

22. Vorrichtung zur Unterstützung bei der respiratorischen Beatmung (10) nach einem der vorangehenden Ansprüche,
**dadurch gekennzeichnet, dass** der Verteilerblock (50) Steuereinrichtungen (122) umfasst, die das Öffnen und das Schließen eines Ausatmungsventils (120) steuern, die sowohl in der Einatmungsleitung (14) eines einfachen Kreises (118) als auch in der Ausatmungsleitung (130) eines Doppelkreises (132) angeordnet sein können.

23. Vorrichtung zur Unterstützung bei der Beatmung (10) nach dem vorangehenden Anspruch, **dadurch gekennzeichnet, dass** für den Fall, dass sie einen Doppelschaltkreis (132) umfasst, der Ausatmungsschieber (120) in einem Modul (134) eingerichtet ist, das an dem Verteilerblock (50) befestigt ist und eine Leitung (124) umfasst, so dass die Steuereinrichtung (122) mit dem Ausatmungsschieber (120) verbunden ist.

24. Vorrichtung zur Unterstützung bei der Beatmung (10) nach einem der Ansprüche 22 und 23, **dadurch gekennzeichnet, dass** die Steuereinrichtungen (122) die Erzeugung eines positiven Ausatmungsdruckes an dem Ausatmungsventil (120) ermöglichen, und dass der positive Ausatmungsdruck durch einen Ventilator erzeugt wird.

25. Vorrichtung zur Unterstützung bei der Beatmung (10) nach einem der vorangehenden Ansprüche, **dadurch gekennzeichnet, dass** der Verteilerblock (50) Messeinrichtungen (126) umfasst, die den Durchsatz von zirkulierendem Gas bestimmen, wenn man sich in der Ausatmungsphase in der Einatmungsleitung (14) eines einfachen Kreises (118) oder in der Ausatmungsleitung (130) eines Doppelkreises (132) befindet.

26. Vorrichtung zur Unterstützung bei der Beatmung (10) nach dem vorangehenden Anspruch, **dadurch gekennzeichnet, dass** bei Vorliegen eines Doppelkreises (132) ein Modul (134) vorgesehen ist, das an dem Verteilerblock (50) befestigt ist und das wenigstens eine Leitung (129) umfasst, die die Messeinrichtungen (126) mit der Ausatmungsleitung (130) verbindet.
